# EUROPEAN PATENT APPLICATION

(11) **EP 4 814 070 A1**
(43) Date of publication of application: **30.09.2026**
(21) Application number: 24892868.1
(22) Date of filing: 30.07.2024
(51) Int. Cl.: C09D 143/02, C08F 230/02, G03F 7/027

(54) **NEW-TYPE ANTIFOULING DEVELOPING COATING MATERIAL, AND PREPARATION METHOD THEREFOR AND USE THEREOF**

(30) Priority: 24.11.2023 CN 202311578586; 28.06.2024 CN 202410861302
(71) Applicant: Suzhou Silver Mars New Materials Technology Co., Ltd, Suzhou, Jiangsu 215000 (CN)
(72) Inventor: XUE, Bin, Suzhou, Jiangsu 215000 (CN); CAO, Yi, Suzhou, Jiangsu 215000 (CN); YANG, Xin, Suzhou, Jiangsu 215000 (CN)
(74) Representative: HGF
(86) International application number: PCT/CN2024/108427
(87) International publication number: WO 2025/107704

(57) **Abstract**

A new-type antifouling developing coating material, and a preparation method therefor and the use thereof. The antifouling developing coating material has a structure represented by formula I. Compared with a traditional developing coating, the antifouling developing coating material has higher medical safety, can ensure the stability and durability of a developing effect, and has a wider range of application.

## Description

### TECHNICAL FIELD

The present application relates to the technical field of medical materials, for example, a novel antifouling radiopaque coating material, a preparation method therefor and use thereof.

### BACKGROUND

Medical radiopaque coatings play a critical role in medical imaging. Advancing medical imaging requires higher safety and better radiopaque efficacy from the radiopaque coatings. Medical radiopaque coatings must possess excellent antifouling capabilities and can effectively resist the attachment of bacterial and protein to prevent bacterial infections and cross-infections, while maintaining a clean and transparent surface to ensure clear and accurate imaging. Additionally, these medical radiopaque coatings need to deliver distinct and precise radiopaque performance.

### I. Importance of Antifouling Performance and Research Progress

Conventional radiopaque coatings provide radiopaque functionality, but their antifouling capabilities are limited. With the increasing use of medical devices, there is a growing need for coatings that combine radiopacity with antifouling properties. To meet this need, antifouling components have been introduced into coating systems to improve resistance to fouling. The antifouling mechanism primarily involves the hydrophilicity and lipophilicity of material surfaces. Hydrophilic surfaces have large contact angles with water, preventing moisture and impurities from adhering. Lipophilic surfaces resist the adhesion of organic matter or oils, reducing the adsorption of biomolecules.

In recent years, significant progress has been made in the research of medical antifouling coatings. Researchers have modified surface properties to develop coatings with superior antifouling performance. For example, superhydrophobic polymer materials are introduced to create superhydrophobic effects on the surfaces, enabling efficient self-cleaning and antifouling. Meanwhile, biomimetic designs have been employed to create special surface structures, such as convex and concave micro- or nano-structures and honeycomb-like structure, reducing contact areas and minimizing contaminant adhesion. Bioactive molecules embedded in the coatings form "antimicrobial zones" that inhibit microbial proliferation, further enhancing antifouling effectiveness.

### II. Importance of Radiopaque Performance and Research Progress

The radiopaque performance of medical antifouling radiopaque coatings critically impacts the clarity of medical images and the accuracy of diagnoses. These radiopaque coatings must be sensitive to X-rays to ensure effective imaging results under X-ray exposure. Radiopaque technology has been widely used in medical imaging, where clear images are formed based on the imaging efficiency of photosensitive materials. X-ray contrast is generally achieved through the absorption and scattering of X-rays by radiopaque materials. The radiopaque coatings play a key role in this process by absorbing and scattering X-rays to form images.

Research on medical radiopaque coatings has evolved alongside advancements in medical imaging technology. Researchers have improved the radiopaque agents and chemical compositions of radiopaque coatings to enhance radiopaque efficiency and image quality. Meanwhile, new technologies such as nanotechnology and quantum dots have been introduced to enhance the sensitivity and selectivity of these coatings.

### III. Challenges and Research Directions in Combining Radiopacity and Antifouling

Maintaining radiopaque performance while achieving excellent antifouling performance is a great challenge. Interactions between radiopaque agents and antifouling agents may reduce radiopaque efficiency. The selection of coating materials and the design of their structures require greater precision to achieve an optimal balance between radiopaque and antifouling performance. To address this challenge, researchers can explore diverse combinations of radiopaque agents and antifouling agents, optimizing structures and chemical compositions of the coatings. At the same time, nanotechnology and surface engineering can be leveraged to construct multifunctional radiopaque coatings, synergistically enhancing both radiopaque and antifouling capabilities.

In summary, medical radiopaque coatings are of paramount importance in medical imaging. Through ongoing research to improve radiopaque performance and antifouling capabilities, it is anticipated that more advanced, safer, and more reliable radiopaque coatings will be developed, providing better technical support for medical diagnosis and treatment. Furthermore, research aimed at integrating radiopaque and antifouling functions holds promise for achieving new breakthroughs in the development of medical radiopaque materials.

### SUMMARY

The following is a summary of the subject matter described in detail in the present application. The summary is not intended to limit the scope of the claims.

The present application provides a novel antifouling radiopaque coating material, a preparation method therefor and use thereof. In the present application, safer and more reliable radiopaque coatings are developed with more excellent performance, providing better technical support for medical diagnosis and treatment.

In an aspect, the present application provides a novel antifouling radiopaque coating material, where the antifouling radiopaque coating material has a structure represented by Formula I: 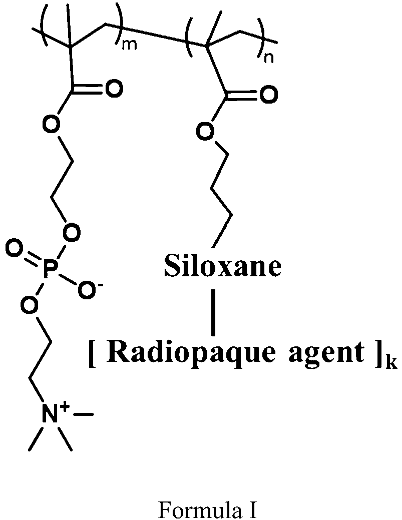 where m = 1 - 40 (for example, 1, 2, 3, 5, 8, 10, 13, 15, 18, 20, 22, 25, 28, 30, 32, 34, 36, 38 or 40), n = 1 - 40 (for example, 1, 2, 3, 5, 8, 10, 13, 15, 18, 20, 22, 25, 28, 30, 32, 34, 36, 38 or 40), and k = 1 - 3 (for example, 1, 2 or 3).

The antifouling radiopaque coating material described in the present application combines antifouling and radiopaque properties through covalent bonding technology, ensuring the stability of the material and the stability of the antifouling and radiopaque performance. Therefore, the material exhibits outstanding performance in both the antifouling capability and the radiopaque efficacy, thereby providing the material with broad application prospects in the field of medical imaging and offering strong support for the safety of medical devices and the accuracy of medical imaging.

In some optional embodiments, the radiopaque agent is one or a mixture of at least two selected from the group consisting of iohexol, iohexol hydrolysate, iopromide, meglumine diatrizoate, sodium diatrizoate, lipiodol, and iodixanol.

In a more optional embodiment, the radiopaque agent is iohexol or iohexol hydrolysate.

In some optional embodiments, the radiopaque agent and the siloxane are linked via a linking group.

In some optional embodiments, the radiopaque agent and the siloxane are linked by forming Si-O-Si linkage.

In some optional embodiments, the antifouling radiopaque coating material has a structure represented by Formula II: 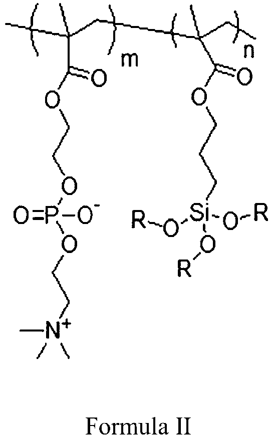 where m = 1 - 40 (for example, 1, 2, 3, 5, 8, 10, 13, 15, 18, 20, 22, 25, 28, 30, 32, 34, 36, 38 or 40), n = 1 - 40 (for example, 1, 2, 3, 5, 8, 10, 13, 15, 18, 20, 22, 25, 28, 30, 32, 34, 36, 38 or 40), k = 1 - 3 (for example, 1, 2 or 3), R is selected from C1 - C5 alkyl or C1 - C5 alkylsilyl, and L is

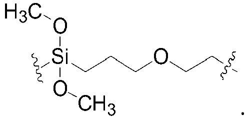

In some optional embodiments, m:n = 1:1 - 4:1, for example, 1:1, 2:1, 3:1 or 4:1. In the present application, when the ratio of m:n is within the range of 1:1 to 4:1, by adjusting the molar ratio of a modified phosphorylcholine polymer to a barium salt, the polymer material that meets the requirements for radiopaque and antifouling properties can be obtained.

In some optional embodiments, m:n = 3:1.

In another aspect, the present application provides a method for preparing the novel antifouling radiopaque coating material described above. The preparation method includes the following step.

A radiopaque agent modified with a siloxane reagent and a siloxane-modified phosphorylcholine polymer are subjected to hydrolysis and polycondensation to obtain the novel antifouling radiopaque coating material.

Optionally, the siloxane reagent is selected from

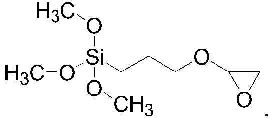

Optionally, the molar ratio of the radiopaque agent modified with the siloxane reagent to the siloxane-modified phosphorylcholine polymer is 40:1 - 10:1, for example, 40:1, 38:1, 35:1, 33:1, 30:1, 28:1, 25:1, 20:1, 18:1, 15:1, 13:1 or 10:1.

Optionally, the hydrolysis and polycondensation of the radiopaque agent modified with the siloxane reagent and the siloxane-modified phosphorylcholine polymer is carried out at room temperature, and the hydrolysis and polycondensation is carried out for 12 - 48 hours (h), for example, 12 h, 14 h, 16 h, 18 h, 20 h, 24 h, 28 h, 30 h, 33 h, 35 h, 38 h, 40 h, 44 h or 48 h, optionally, 24 h.

Optionally, a solvent used in the hydrolysis and polycondensation of the radiopaque agent modified with the siloxane reagent and the siloxane-modified phosphorylcholine polymer is a mixture of methanol and water, a mixture of isopropanol and water, or a mixture of DMSO and water.

Optionally, the radiopaque agent modified with the siloxane reagent is prepared through the following preparation method.

A radiopaque agent and a siloxane reagent are subjected to a reaction in a mixed solvent to obtain the radiopaque agent modified with the siloxane reagent.

Optionally, the molar ratio of the radiopaque agent to the siloxane reagent is 1:1 - 3:1, for example, 1:1, 1.5:1, 1.8:1, 2:1, 2.3:1, 2.5:1, 2.8:1 or 3:1.

Optionally, the reaction of the radiopaque agent and the siloxane reagent is carried out at room temperature, and the reaction is carried out for 3 - 12 h, for example, 3 h, 5 h, 8 h, 10 h, 11 h or 12 h, optionally 6 h.

Optionally, the mixed solvent is a mixture of methanol and water, a mixture of isopropanol and water, or a mixture of DMSO and water, and the volume ratio of the two solvents in the mixed solvent may be 1:1.

Optionally, the siloxane-modified phosphorylcholine polymer has the following structure: 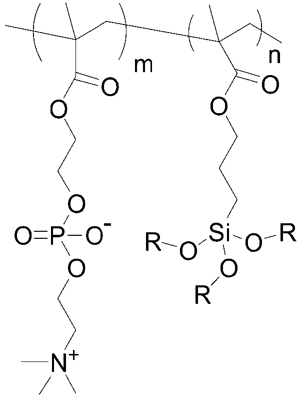 where m = 1 - 40 (for example, 1, 2, 3, 5, 8, 10, 13, 15, 18, 20, 22, 25, 28, 30, 32, 34, 36, 38 or 40), n = 1 - 40 (for example, 1, 2, 3, 5, 8, 10, 13, 15, 18, 20, 22, 25, 28, 30, 32, 34, 36, 38 or 40), k = 1 - 3 (for example, 1, 2 or 3), and R is selected from C1 - C5 alkyl or C1 - C5 alkylsilyl.

Optionally, the siloxane-modified phosphorylcholine polymer is prepared through the following preparation method.

2-methacryloyloxyethyl phosphorylcholine and a siloxane compound are subjected to a reaction to obtain the siloxane-modified phosphorylcholine polymer.

Optionally, the siloxane compound is methacryloxypropyltris(trimethylsiloxy)silane and/or 3-(methacryloyloxy)propyltrimethoxysilane.

Optionally, the molar ratio of 2-methacryloyloxyethyl phosphorylcholine to the siloxane compound is 40:1 - 10:1, for example, 40:1, 38:1, 35:1, 33:1, 30:1, 28:1, 25:1, 20:1, 18:1, 15:1, 13:1 or 10:1.

Optionally, the reaction of 2-methacryloyloxyethyl phosphorylcholine and the siloxane compound is carried out in the presence of 4-cyano-4-(thiobenzoylthio)pentanoic acid.

Optionally, the reaction of 2-methacryloyloxyethyl phosphorylcholine and the siloxane compound is carried out in the presence of an initiator.

Optionally, the initiator is selected from azobisisobutyronitrile.

Optionally, the reaction of 2-methacryloyloxyethyl phosphorylcholine and the siloxane compound is carried out at a temperature of 60 °C - 70 °C, for example, 60 °C, 63 °C, 65 °C, 68 °C or 70 °C, and the reaction is carried out for 12 - 48 h, for example, 12 h, 14 h, 16 h, 18 h, 20 h, 24 h, 28 h, 30 h, 33 h, 35 h, 38 h, 40 h, 44 h or 48 h, optionally, 24 h.

Optionally, the reaction of 2-methacryloyloxyethyl phosphorylcholine and the siloxane compound is carried out in a solvent, and the solvent is selected from *n*-propanol.

In another aspect, the present application provides the use of the novel antifouling radiopaque coating material described above in coating a substrate surface.

Optionally, the substrate surface includes a silicon substrate surface, a glass substrate surface, a metal substrate surface, or a polymer substrate surface.

The material of the present application can be applied to a variety of substrate surfaces, including glass substrates, metal substrates, and various plastic and polymer substrates (such as silicone rubber, polyethylene, polypropylene, polyvinyl chloride, polystyrene or polyurethane).

In the present application, the radiopaque functional monomer and the antifouling functional polymer in the novel antifouling radiopaque coating material are linked via a silicon-oxygen moiety, and the silicon-oxygen compound can be bonded firmly to the substrate, thereby ensuring the stability and safety of the coating material of the present application on the substrate. Particularly, in the case of a silicon-based substrate, the silicon-oxygen compound can be incorporated into the substrate material or even covalently bonded to the substrate material, thereby further ensuring safety.

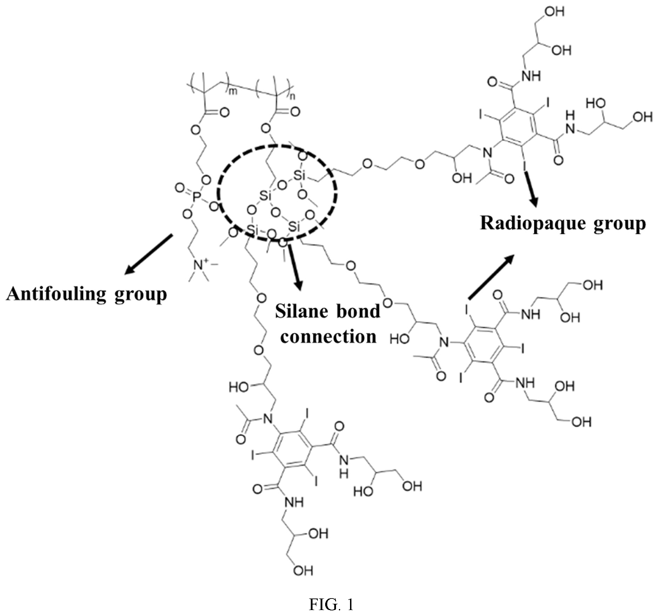

The present application provides a design and preparation approach for a novel antifouling radiopaque coating applicable to any substrate surface (preferably a silicon-based substrate surface). The structural design principle of the molecular configuration of the coating is illustrated in FIG. 1. The molecular structure of the novel antifouling radiopaque coating material involved in the present application mainly includes two parts: a radiopaque functional monomer and an antifouling functional polymer. The radiopaque functional monomer employs a compound capable of covalently bonding to phosphorylcholine, such as an intermediate molecule obtained by reacting an iodine-containing compound (for example, iohexol hydrolysate) with epoxy silane. The radiopaque functional monomer possesses excellent X-ray absorption performance and can enable accurate and clear imaging in medical imaging, thereby providing reliable image information for medical imaging. The radiopaque functional monomer is tightly integrated with the antifouling functional polymer of modified phosphorylcholine, which also contains silane groups, through silane hydrolysis and polymerization, forming a composite structure while retaining their respective characteristics. By introducing the antifouling functional unit, the coating is endowed with outstanding antifouling performance and enabled to effectively inhibit adhesion of protein and bacteria, thereby preventing bacterial infections and cross-infections. In scenarios imposing high hygiene standards, such as medical devices and catheters, the achieved antifouling performance is of great significance, ensuring the hygienic safety of medical devices. Through the synergistic action of the radiopaque functional monomer and the antifouling functional unit, the novel antifouling radiopaque coating forms a product containing silane groups. The covalent bonding approach grants the coating exceptional stability on the silicon substrate surface and prevents the coating from leaching or detaching over time, thereby significantly enhancing the durability and reliability of the radiopaque coating. Meanwhile, owing to the universality of silane groups, the coating can be bonded to a variety of substrate surfaces, including glass substrates, metal substrates, and various plastic and polymer substrates (such as silicone rubber, polyethylene, polypropylene, polyvinyl chloride, polystyrene or polyurethane), thereby providing broad applicability and flexibility for the application fields of the radiopaque coating.

The design concept of the antifouling radiopaque coating material in the present application ingeniously combines radiopaque and antifouling functions. By rationally selection of monomers and through covalent bonding, the coating is endowed with both a radiopaque capability and antifouling performance. The design concept is universal and practical, offering a new direction for the research, development, and application of medical radiopaque materials in medical imaging and bringing further progress and breakthroughs to the field of medical diagnosis and treatment.

In another aspect, the present application provides an antifouling radiopaque coating, where the raw material for preparing the antifouling radiopaque coating includes the novel antifouling radiopaque coating material described above.

The radiopaque functional monomer and the antifouling functional polymer are mixed at a mass ratio of 2:1, 1:1 or 1:2 in the novel antifouling radiopaque coating, and during the preparation of the antifouling radiopaque coating, the antifouling radiopaque coating material needs to be dissolved in a solvent (for example, n-propanol) and has a concentration of 400 mg/mL.

In another aspect, the present application provides the use of the novel antifouling radiopaque coating material described above in a medical device, a medical material, an optical lens or industrial printing.

The coating material of the present application can be applied as an antifouling radiopaque functional coating for medical devices, endowing the coated object with both antifouling and X-ray imaging capabilities. The novel antifouling radiopaque coating of the present application can also be further applied as an antifouling radiopaque functional coating in optical lens coatings and industrial printing.

Compared with the related art, the present application has the following beneficial effects.

The antifouling coating prepared from the novel antifouling radiopaque coating material of the present application on medical devices exhibits excellent antifouling performance, representing a significant improvement in antifouling compared with the conventional radiopaque coating. The coating contains a unique antifouling component that can reduce bacteria and protein adhesion, thereby helping to reduce the risk of contamination and infection. This characteristic is crucial for medical safety, ensuring hygiene and safety during the use of medical devices. The improvement of the radiopaque coating makes it suitable for various medical scenarios, holding particular significance for equipment such as operating rooms and medical catheters that demand high hygiene standards.

In addition, the novel antifouling radiopaque coating demonstrates outstanding radiopaque performances. Compared with the conventional radiopaque coating, the novel antifouling radiopaque coating achieves covalent bonding to the substrate and thus is prevented from leaching and reduces release from the substrate, thereby ensuring the stability and durability of the radiopaque performance. This feature may also contribute to eliminating potential adverse effects caused by the radiopaque coating, and improving the safety of the medical radiopaque coatings. This is beneficial to patient health and medical experience.

Furthermore, the novel antifouling radiopaque coating of the present application has wide applicability. Through silane bond polymerization, the coating can achieve covalent bonding to any silicon-based substrate, including glass, silicone sheets, marble, and various other silicon substrate surfaces, making it widely applicable to different clinical and laboratory scenarios to meet the needs of diverse users. The preparation method of the above-mentioned coating is simpler and costs less compared with the conventional coating, providing a feasible pathway for the large-scale application of radiopaque coatings.

In summary, the innovation of the novel antifouling radiopaque coating lies in its successful integration of antifouling and radiopaque functions, addressing a key challenge in this field. The conventional radiopaque coating often compromises between radiopaque efficacy and antifouling performance, whereas the novel coating ingeniously combines the radiopaque efficacy with the antifouling performance through covalent bonding technology, which provides a new direction for the research and development of medical radiopaque materials and offers a more innovative solution. The new antifouling radiopaque coating exhibits exceptional performance in antifouling properties, radiopaque performances, universality, and innovation. Its superior performance endows it with broad application prospects in the field of medical imaging, providing strong support for the safety of medical devices and the accuracy of medical imaging. Through continuous research and innovation, it is believed that the novel antifouling radiopaque coating will bring further progress and breakthroughs to medical diagnosis and treatment.

Other aspects can be understood after the drawings and the detailed description are read and understood.

### BRIEF DESCRIPTION OF DRAWINGS

The drawings are used to provide a further understanding of technical solutions herein, constitute part of the specification, serve to explain the technical solutions herein in conjunction with embodiments of the present application, and do not constitute limitations on the technical solutions herein.
FIG. 1 shows the design principle of a novel antifouling radiopaque coating.
FIG. 2 shows nuclear magnetic resonance (NMR) spectrum of a radiopaque compound used for preparing the novel antifouling radiopaque coating.
FIG. 3 shows X-ray photoelectron spectroscopy (XPS) results of the radiopaque compound used for preparing the novel antifouling radiopaque coating.
FIG. 4 shows the nuclear magnetic resonance spectrum of a comparative radiopaque molecule obtained from reaction of sodium diatrizoate with an aminosilane..
FIG. 5 shows the nuclear magnetic resonance (NMR) spectrum of a comparative radiopaque molecule obtained by the combination of ioversol with 3-glycidyloxypropyltrimethoxysilane.
FIG. 6 shows the nuclear magnetic resonance (NMR) spectrum of a radiopaque molecule obtained by the combination of iohexol with isocyanate silane through an ester bond.
FIG. 7A shows the radiopaque performance of the novel antifouling radiopaque coating.
FIG. 7B shows the results of quantitative analysis of the grayscale value of the radiopaque performance of the novel antifouling radiopaque coating.
FIG. 8A is a fluorescence microscopy image showing the protein repellent property of the novel antifouling radiopaque coating.
FIG. 8B shows the results of fluorescence quantitative analysis of the protein repellent property of the novel antifouling radiopaque coating.
FIG. 9A is a SEM image showing the results of the bacterial adhesion test of the novel antifouling radiopaque coating.
FIG. 9B shows the results of quantitative analysis of bacterial colonies on the novel antifouling radiopaque coating to evaluate resistance to bacterial adhesion.
FIG. 10A shows the test results of the radiopaque performance of the novel antifouling radiopaque coating in rat and porcine tissues.
FIG. 10B shows the results of the quantitative analysis of the radiopaque performance of the novel antifouling radiopaque coating inside rat tissues.
FIG. 10C shows the results of the quantitative analysis of the radiopaque performance of the novel antifouling radiopaque coating inside porcine tissues.

### DETAILED DESCRIPTION

The technical solutions in embodiments of the present application will be described clearly and completely via the specific embodiments and drawings in the present application. Apparently, the embodiments described below are part, not all, of the embodiments of the present application. Based on the embodiments of the present application, all other embodiments obtained by those of ordinary skill in the art without creative work are within the scope of the present application.

In the description of the present application, it should be noted that terms such as "upper", "lower", "inner", "outer", "front end", "rear end", "both ends", "one end", "the other end", and the like indicating orientation or positional relationships are used solely for the convenience of describing the present application and simplifying the description, are not intended to indicate or imply that the referenced device or element must have a specific orientation or be constructed or operated in a specific orientation, and therefore should not be construed as limitations on the present application. Furthermore, terms such as "first" and "second" are used for descriptive purposes only and should not be understood as indicating or implying relative importance.

In the description of the present application, it should be noted that, unless otherwise expressly specified and limited, the terms "installed", "provided with", "connected", and the like should be interpreted in a broad sense. For example, "connected" may refer to a fixed connection, a detachable connection, or an integral connection; it may also be an indirect connection via an intermediate medium, or it may refer to internal communication between two elements. For those of ordinary skill in the art, specific meanings of the preceding terms in the present application can be construed based on their specific context.

### Example 1 Synthesis example of modified phosphorylcholine

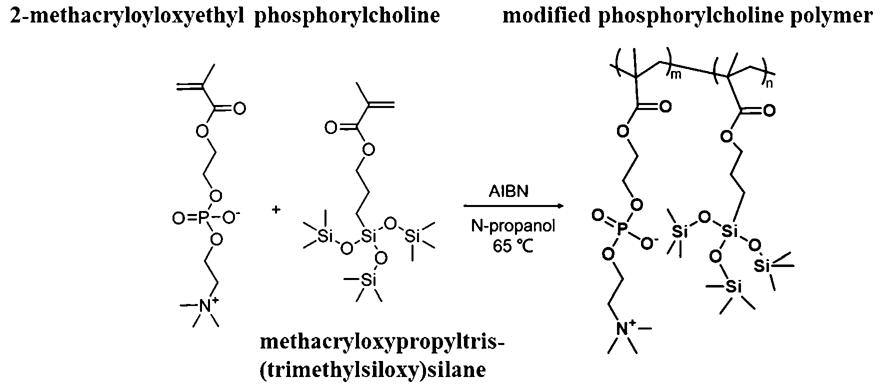

2-methacryloyloxyethyl phosphorylcholine (3 g), 4-cyano-4-(thiobenzoylthio)pentanoic acid (3 g), and azobisisobutyronitrile (0.16 g) were dissolved in n-propanol (100 mL). Under nitrogen protection, the mixture was slowly heated to 65 °C and stirred for 24 h. Then, methacryloxypropyltris(trimethylsiloxy)silane (8 mL) was added, and the reaction was continued for another 24 h. The resulting n-propanol solution was added into diethyl ether (200 mL), precipitated, filtered, and dried to obtain the phosphorylcholine polymer.

### Example 2 Preparation example of a novel antifouling radiopaque coating material

a) Iohexol hydrolysate (5 g) was dissolved in 10 mL of a methanol/water mixed solvent (1:1). Potassium hydroxide (1.67 g) was then added, and the mixture was stirred and ultrasonicated at room temperature until fully dissolved, yielding a pale yellow solution. Substance 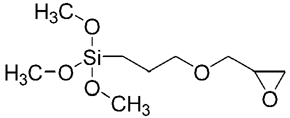 (2 g) was dissolved in 10 mL of a methanol/water mixed solvent, then mixed with the pale yellow solution, and stirred overnight at room temperature. The reaction product was then extracted from the methanol/water solvent into an organic phase using ethyl acetate, and the extraction was repeated five times. The reaction mixture was evaporated using a rotary evaporator to remove the organic solvent and then subjected to dialysis and liquid chromatography purification to obtain a silane-modified radiopaque functional monomer C₂₇H₄₄I₃N₃O₁₃Si.
b) The radiopaque functional monomer prepared from a) and the modified phosphorylcholine were mixed at different molar ratios (that is, at 2:1, 1:1, and 1:2) in the methanol/water mixed solvent (having a methanol/water volume ratio of 1:1). The mixture was fully stirred at room temperature for 6 h to allow sufficient hydrolysis and condensation with the alkylsilyl of the antifouling molecule of modified phosphorylcholine. After purification by organic solvent extraction, rotary evaporation, and other means, an antifouling radiopaque polymer was obtained (FIG. 1).

### Example 3 Tests for the basic characterization of the compound of the present application

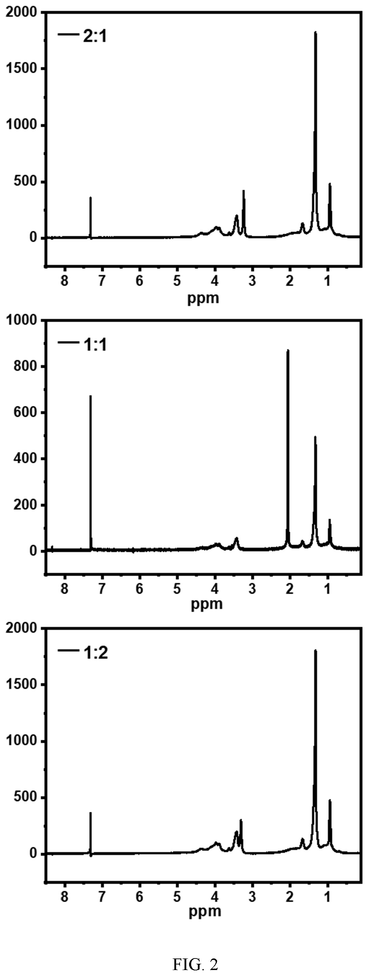

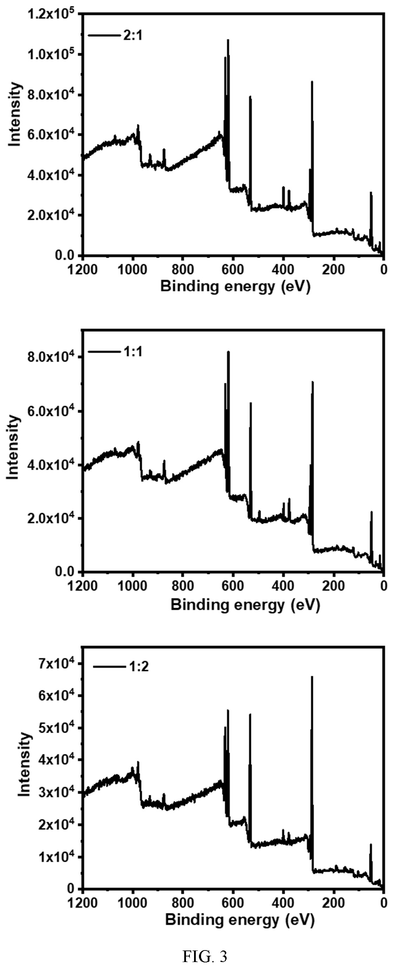

To determine whether the prepared novel antifouling radiopaque material was successfully synthesized and its elemental composition, proton nuclear magnetic resonance (¹HNMR, deuterated chloroform) and X-ray photoelectron spectroscopy (XPS) tests were carried out on all samples. As shown in FIG. 2, the ¹H NMR spectrum shows vibration peaks at 7.3 ppm and 3.55 ppm, which are contributed by the iodophenyl group in the radiopaque monomer and the siloxane group in the antifouling molecule, respectively, demonstrating the successful synthesis of the antifouling radiopaque polymer material. As shown in FIG. 3, the XPS spectrum shows peaks at 285 eV, 532 eV, 400 eV, and 620 eV, which are contributed by elements C, O, N, and I, respectively. Moreover, as the proportion of the iodine-containing radiopaque monomer increased, the content of element I in the samples increased from 2.59% to 4.2%, demonstrating the successful polymerization of the iodine-containing radiopaque monomer with the antifouling molecule of modified phosphorylcholine.

### Example 4

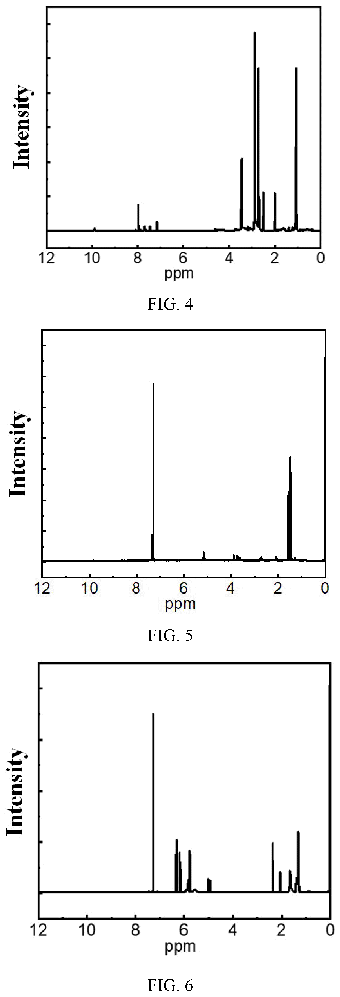

a) Sodium diatrizoate (5 g) was dissolved in 10 mL of an ethanol/water mixed solvent (1:1). O-benzotriazole-tetramethyluronium hexafluorophosphate (0.85 g) and triethylamine (600 µL) were then added, and the mixture was stirred and ultrasonicated at room temperature until fully dissolved, yielding a colorless solution. (3-aminopropyl)triethoxysilane (2 g) was dissolved in 10 mL ethanol, then mixed with the colorless solution, and stirred overnight at room temperature. The reaction product was then extracted into an organic phase using ethyl acetate, and the extraction was repeated five times. The reaction mixture was evaporated using a rotary evaporator to remove the organic solvent and then subjected to dialysis and liquid chromatography purification to obtain a radiopaque molecule having the combination of sodium diatrizoate with aminosilane. The NMR data of the resulting molecule are shown in FIG. 4.
b) The radiopaque molecule obtained from a) and modified phosphorylcholine were mixed at different ratios (that is, at 2:1, 1:1, and 1:2) in the methanol/water solvent (methanol/water volume ratio of 1:1), and subjected to hydrolysis and polymerization to obtain an antifouling radiopaque polymer.

### Example 5

a) Ioversol (5 g) was dissolved in 10 mL of a methanol/water mixed solvent (1:1). Potassium hydroxide (1.67 g) was then added, and the mixture was stirred and ultrasonicated at room temperature until fully dissolved, yielding a pale yellow solution. 3-glycidyloxypropyltrimethoxysilane (2 g) was dissolved in 10 mL of a methanol/water mixed solvent, then mixed with the pale yellow solution, and stirred overnight at room temperature. The reaction product was then extracted from the methanol/water solvent into an organic phase using ethyl acetate, and the extraction was repeated five times. The reaction mixture was evaporated using a rotary evaporator to remove the organic solvent and then subjected to dialysis and liquid chromatography purification to obtain a radiopaque functional monomer, whose NMR data are shown in FIG. 5.
b) The radiopaque molecule obtained from a) and modified phosphorylcholine were mixed at different ratios (that is, at 2:1, 1:1, and 1:2) in the methanol/water solvent (methanol/water volume ratio of 1:1), and subjected to hydrolysis and polymerization to obtain an antifouling radiopaque polymer.

### Comparative Example 1

Iohexol (5 g) was dissolved in 10 mL of a methanol/water mixed solvent (1:1). Potassium hydroxide (1.67 g) was then added, and the mixture was stirred and ultrasonicated at room temperature until fully dissolved, yielding a pale yellow solution. 3-isocyanatopropyltriethoxysilane (2 g) was dissolved in 10 mL of a methanol/water mixed solvent, then mixed with the pale yellow solution, and stirred overnight at room temperature. The reaction product was then extracted from the methanol/water solvent into the organic phase using ethyl acetate, and the extraction was repeated five times. The reaction mixture was evaporated using a rotary evaporator to remove the organic solvent and then subjected to dialysis and liquid chromatography purification to obtain a radiopaque functional monomer obtained by the reaction of isocyanate with alcohol hydroxyl groups, whose NMR data are shown in FIG. 6. Since the connection mode is changed from the silane bond to the ester bond and the ester bond is unstable and prone to hydrolysis, the nuclear magnetic peaks are shifted.

### Example 6 Tests on the radiopaque performances of antifouling radiopaque polymer coatings synthesized with different proportions of components of the present application

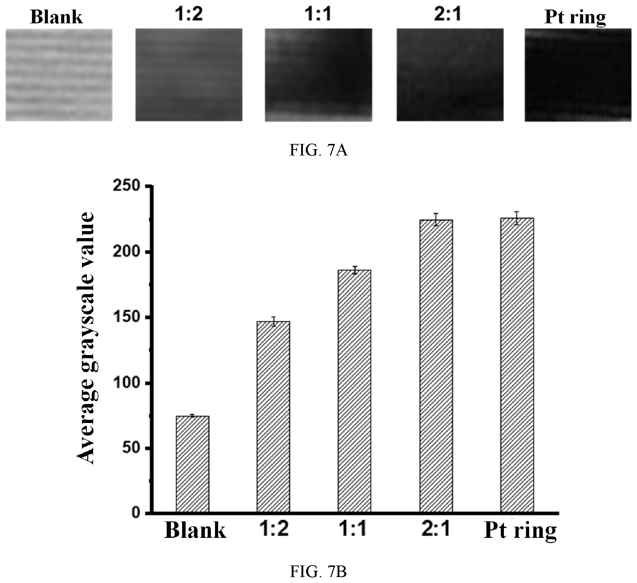

The radiopaque performances of the antifouling radiopaque coatings were tested by using a medical X-ray irradiator. The control groups were a blank negative control and a positive control of a metal platinum ring. The ratios of different raw material components were adjusted to optimize the radiopaque performance of the coating. As shown in FIGS. 7A and 7B, the mass ratios of the radiopaque molecule iohexol to the antifouling molecule of modified phosphorylcholine in the experimental groups were 2:1, 1:1, and 1:2, respectively. As can be seen from FIG. 7A, the radiopaque performance of the coating was significantly enhanced with the increase in the proportion of iohexol, and when the proportion of iohexol was high enough, the radiopaque performance of the coating was comparable to that of metal platinum, indicating that the antifouling radiopaque coating has excellent radiopaque performance and can enable clear imaging under X-ray irradiation. The grayscale values of the radiopaque performances of the coatings were quantitatively analyzed using ImageJ software (FIG. 7B). The control groups were a blank negative control and a positive control of a metal platinum ring. As shown in FIG. 7B, the grayscale value of the radiopaque performance was directly proportional to the content of iohexol. When the component ratio was 4:1, the grayscale value was the largest, and the coating exhibited the best radiopaque performance, demonstrating the radiopaque performance comparable to that of the metal platinum ring.

### Example 7 Tests on the protein repellent property of the antifouling radiopaque polymer coating of the present application

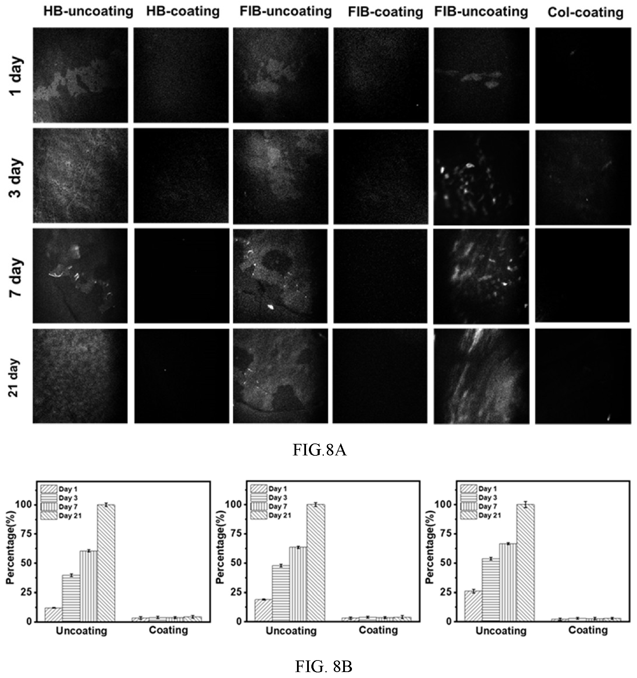

In terms of resisting protein adsorption, the adsorption capacities of fibrin (FIB), serum protein (HB), and collagen (Col) on the antifouling radiopaque coating were measured and characterized. The ratio of the radiopaque molecule to the antifouling molecule in the antifouling radiopaque coating molecule used was 1:2. The protein molecules used were modified with fluorescent molecules in advance. The adsorption test was carried out for 21 days. The adsorption of various molecules on the coating was observed using a fluorescence microscope (model: FV3000-Olympus; production place: Japan; manufacturer: Olympus) on Days 1, 3, 7, and 21 (labeled as Day 1, Day 3, Day 7, and Day 21 in FIG. 8B). The control groups were blank controls without the antifouling radiopaque coating (the blank controls were represented by "Uncoating" in FIGS. 8A and 8B whereas the coating groups were represented by "Coating"). As shown in FIG. 8A, the coating exhibited an excellent anti-protein adsorption capability compared with the blank controls. Serum proteins were hardly adsorbed to the coating, and the adsorption of fibrin on the antifouling radiopaque coating was extremely low, whereas the blank control groups were covered with adsorbed protein molecules. Furthermore, the fluorescence quantitative analysis of the total amount of adsorbed molecules (FIG. 8B) was carried out. Compared with the blank control groups, the total amount of proteins adsorbed on the antifouling radiopaque coating was significantly reduced, demonstrating the superior anti-protein adsorption capability of the antifouling radiopaque coating.

### Example 6 Tests on the bacterial repellent property of the antifouling radiopaque polymer coating of the present application

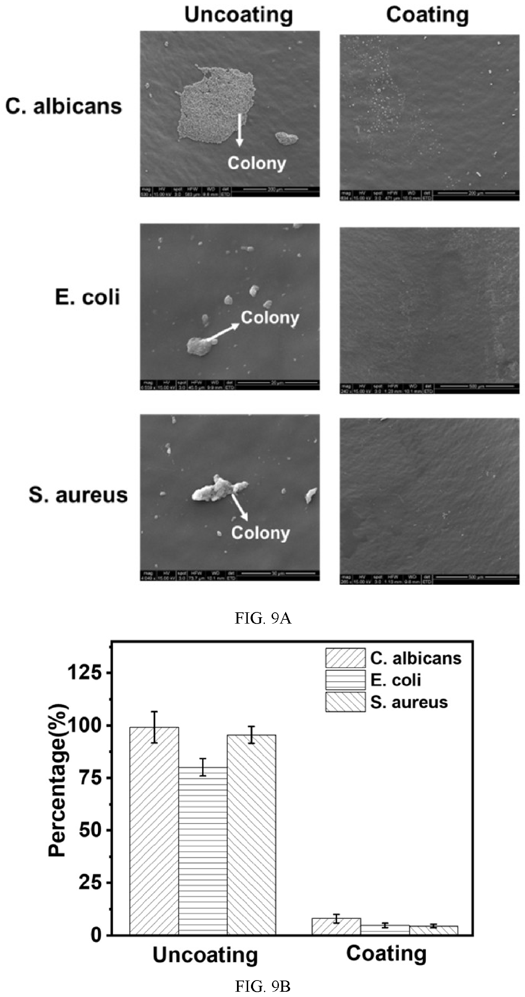

To evaluate resistance to bacterial adhesion, theresistance to bacterial adhesion of the antifouling radiopaque polymer coating was tested and characterized using *Candida albicans* (*C. albicans*), *Escherichia coli* (*E. coli*), and *Staphylococcus aureus* (*S. aureus*). The ratio of the radiopaque molecule to the antifouling molecule in the antifouling radiopaque coating molecule used was 1:2. After the samples were immersed in high-concentration (10⁸/mL) bacterial suspensions of the three strains for two weeks, the samples were removed and air-dried. Bacterial adhesion on the sample surfaces was observed using SEM. The bacterial suspensions contained live bacteria in a dialysate solvent, and the live bacteria were replaced every three days to maintain bacterial activity. As shown in FIG. 9A, after two weeks of exposure to the three different bacterial strains, large visible bacterial colonies were present on the control groups without the antifouling radiopaque coating, whereas no significant bacterial colony growth was observed on the samples coated with the antifouling radiopaque coating. Furthermore, the adhered bacterial colonies were quantitatively analyzed (FIG. 9B). The samples coated with the antifouling radiopaque coating showed significantly reduced bacterial adhesion, with a 95% reduction in the amount of adhered bacteria after two weeks.

### Example 7 Tests on the radiopaque performance of the antifouling radiopaque polymer coating of the present application in rat and porcine tissues

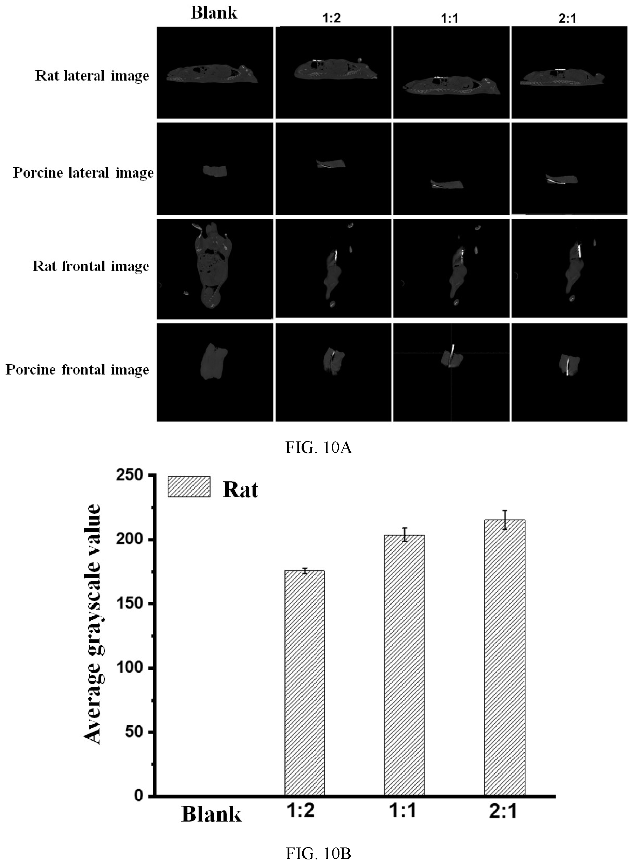

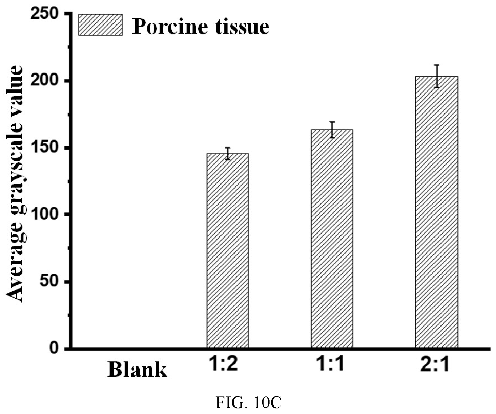

The antifouling radiopaque coating was applied to a silicone catheter with a diameter of 0.5 mm. The catheter was inserted into rat and porcine tissues, and lateral and frontal images of the rat and porcine tissues were taken using a medical X-ray irradiator (manufacturer: XpectVision Technology; model: XVS2530). The control group was a blank negative control. The mass ratios of the radiopaque molecule iohexol to the antifouling molecule of modified phosphorylcholine in the experimental groups were 2:1, 1:1, and 1:2, respectively. As shown in FIG. 10A, the antifouling radiopaque coating exhibited a clear radiopaque performance inside both rat and porcine tissues, producing distinct images under X-ray irradiation. As shown in FIGS. 10B and 10C, the quantitative analysis of the radiopaque region was carried out using ImageJ software, and it is revealed that the radiopaque intensity inside both rat and porcine tissues significantly increased with the proportion of iohexol.

The applicant has stated that although the novel antifouling radiopaque coating material, the preparation method therefor and the use thereof of the present application are described through the preceding embodiments, the present application is not limited to the preceding embodiments, which means that the implementation of the present application does not necessarily depend on the preceding embodiments. It should be apparent to those skilled in the art that any improvements made to the present application and equivalent replacements of raw materials adopted, additions of adjuvant ingredients, selections of specific manners, etc., in the present application all fall within the scope of protection and disclosure of the present application.

## Claims

1. An antifouling radiopaque coating material, which has a structure of Formula I: 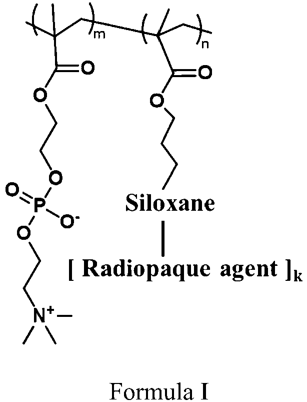 wherein m = 1 - 40, n = 1 - 40, and k = 1 - 3.

2. The antifouling radiopaque coating material according to claim 1, wherein the radiopaque agent is one or a mixture of at least two selected from the group consisting of iohexol, iohexol hydrolysate, iopromide, meglumine diatrizoate, sodium diatrizoate, lipiodol, and iodixanol, optionally iohexol or iohexol hydrolysate.

3. The antifouling radiopaque coating material according to claim 1 or 2, wherein the radiopaque agent and the siloxane are linked via a linking group.

4. The antifouling radiopaque coating material according to any one of claims 1 to 3, wherein the radiopaque agent and the siloxane are linked by forming Si-O-Si linkage.

5. The antifouling radiopaque coating material according to any one of claims 1 to 4, wherein the antifouling radiopaque coating material has the following structure: 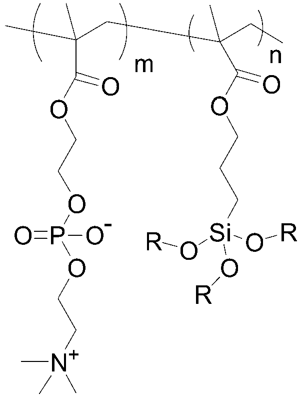 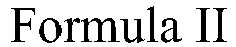 wherein m = 1 - 40, n = 1 - 40, k = 1 - 3, R is selected from C1 - C5 alkyl or C1 - C5 alkylsilyl, and L is

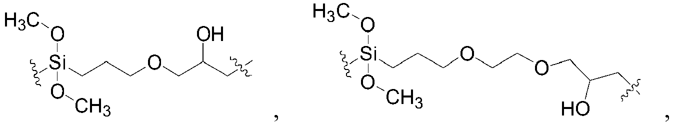

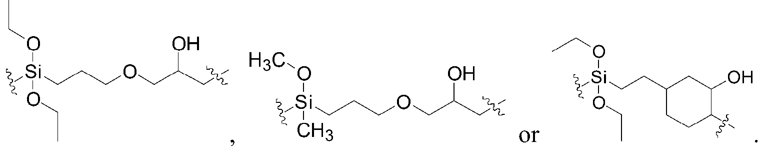

6. The antifouling radiopaque coating material according to any one of claims 1 to 5, wherein m:n = 1:1 - 4:1, and optionally, m:n = 3:1.

7. A method for preparing the antifouling radiopaque coating material according to any one of claims 1 to 6, comprising the following step:
subjecting a radiopaque agent modified with a siloxane reagent and a siloxane-modified phosphorylcholine polymer to hydrolysis and polycondensation to obtain the novel antifouling radiopaque coating material.

8. The preparation method according to claim 7, wherein the siloxane reagent is one or a combination of at least two selected from the group consisting of 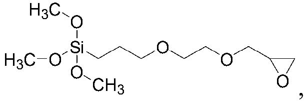 3-glycidoxypropyltriethoxysilane, 3-glycidoxypropylmethyldimethoxysilane, and 2-(3,4-epoxycyclohexyl)ethyltriethoxysilane.

9. The preparation method according to claim 7 or 8, wherein a molar ratio of the radiopaque agent modified with the siloxane reagent to the siloxane-modified phosphorylcholine polymer is 40:1 - 10:1.

10. The preparation method according to any one of claims 7 to 9, wherein the hydrolysis and polycondensation of the radiopaque agent modified with the siloxane reagent and the siloxane-modified phosphorylcholine polymer is carried out at room temperature, and the hydrolysis and polycondensation is carried out for 12 - 48 hours; and
optionally, a solvent used in the hydrolysis and polycondensation of the radiopaque agent modified with the siloxane reagent and the siloxane-modified phosphorylcholine polymer is a mixture of methanol and water, a mixture of isopropanol and water, or a mixture of DMSO and water.

11. The preparation method according to any one of claims 7 to 10, wherein the radiopaque agent modified with the siloxane reagent is prepared through the following preparation method:
subjecting a radiopaque agent and a siloxane reagent to a reaction in a mixed solvent to obtain the radiopaque agent modified with the siloxane reagent;
wherein optionally, a molar ratio of the radiopaque agent to the siloxane reagent is 1:1 - 3:1, optionally 2:1;
optionally, the reaction of the radiopaque agent and the siloxane reagent is carried out at room temperature, and the reaction is carried out for 3 - 12 hours, optionally 6 hours; and
optionally, the mixed solvent is a mixture of methanol and water, a mixture of isopropanol and water, or a mixture of DMSO and water.

12. The preparation method according to any one of claims 7 to 11, wherein the siloxane-modified phosphorylcholine polymer has a structure represented by Formula III: 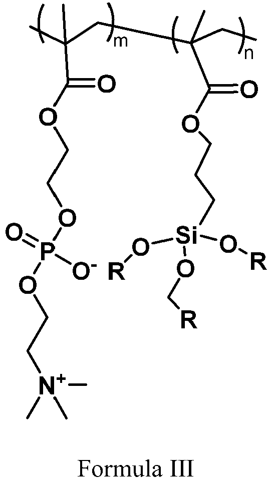 wherein m = 1 - 40, n = 1 - 40, k = 1 - 3, and R is selected from C1 - C5 alkyl or C1 - C5 alkylsilyl.

13. Use of the novel antifouling radiopaque coating material according to any one of claims 1 to 6 in coating a substrate surface;
wherein optionally, the substrate surface comprises a silicon substrate surface, a glass substrate surface, a metal substrate surface, or a polymer substrate surface.

14. An antifouling radiopaque coating, wherein a raw material for preparing the antifouling radiopaque coating comprises the novel antifouling radiopaque coating material according to any one of claims 1 to 6.

15. Use of the novel antifouling radiopaque coating material according to any one of claims 1 to 6 in a medical device, a medical material, an optical lens or industrial printing.
